Europäisches Patentamt

European Patent Office

Office européen des brevets

(19)

(11) Numéro de publication : **0 040 119**
**B1**

# (12) FASCICULE DE BREVET EUROPÉEN

(45) Date de publication du fascicule du brevet :
**15.08.84**

(21) Numéro de dépôt : **81400615.1**

(22) Date de dépôt : **17.04.81**

(51) Int. Cl.³ : **C 10 G 35/095**, B 01 J 29/38,
C 07 C 5/41

(54) **Procédé de déshydrocyclisation des paraffines à très basse pression.**

(30) Priorité : **09.05.80 FR 8010411**

(43) Date de publication de la demande :
**18.11.81 Bulletin 81/46**

(45) Mention de la délivrance du brevet :
**15.08.84 Bulletin 84/33**

(84) Etats contractants désignés :
**BE DE GB IT NL**

(56) Documents cités :
**FR-A- 2 323 664**

(73) Titulaire : **Sté ELF-FRANCE**
**137, rue de l'Université**
**F-75007 Paris (FR)**

(72) Inventeur : **Breysse, Michèle**
**46 Rue Francis de Pressense**
**F-69100 Villeurbanne (FR)**
Inventeur : **Bernard, Jean-René**
**37 Rue des Fleurs**
**F-69360 Serezin du Rhône (FR)**

(74) Mandataire : **Boillot, Marc**
**SOCIETE NATIONALE ELF AQUITAINE Département**
**Propriété Industrielle Tour Aquitaine - Cedex No 4**
**F-92080 Paris la Défense (FR)**

Jouve, 18, rue St-Denis, 75001 Paris, France

## Description

La présente invention concerne un procédé catalytique de production d'hydrocarbures aromatiques à très hauts rendements.

On utilise à très basse pression un catalyseur stable et régénérable par traitement à l'hydrogène, dans deux ensembles de réacteurs en parallèles, l'un étant en charge d'hydrocarbures, l'autre en régénération par l'hydrogène produit par le premier.

Les méthodes habituelles pour réaliser les réactions d'aromatisation des paraffines sont basées sur l'emploi de catalyseurs constitués d'un métal noble sur un support, en particulier des catalyseurs contenant de 0,2 à 0,8 % poids de platine sur un support d'alumine chlorée à 0,2-2 % poids. Pour être suffisamment stables, ces catalyseurs travaillent sous une pression relativement forte (de l'ordre de 30 atm.) en présence d'un excès d'hydrogène (6 moles $H_2$ par mole d'hydrocarbures), de manière à limiter la formation de coke. Malheureusement, ces fortes pression et teneurs en hydrogène limitent thermodynamiquement et cinétiquement les réactions de déshydrocyclisation, tandis que les réactions indésirables d'hydrocraquage sont largement favorisées.

Une amélioration significative de ces catalyseurs a consisté en l'addition d'un second métal au catalyseur, ce qui lui confère une stabilité accrue et la possibilité de travailler à plus basse pression, dans les conditions ou les réactions d'aromatisation sont favorisées. C'est ainsi qu'on utilise maintenant les couples platine-rhénium ou platine-iridium, ou platine-étain, ou platine-germanium, sur le support d'alumine chlorée. Les pressions opératoires ont pu être diminuées à 20 bars environ sans que la durée de cycle ne soit affectée et avec une augmentation notable du rendement en hydrocarbures aromatiques et une diminution des réactions d'hydrocraquage.

Comme les précédents, ces catalyseurs sont utilisés en lit fixe, et après un cycle de quelques mois, ils sont régénérés par combustion du coke suivi d'un traitement par un mélange d'air et de composés chlorés ayant pour but de redisperser les métaux. Après réduction par l'hydrogène, ces catalyseurs se trouvent prêts à l'emploi, avec des propriétés quasi identiques à celles du catalyseur neuf.

Une autre amélioration significative du procédé a consisté à utiliser ces catalyseurs dans des réacteurs à lit mobile. Le catalyseur est injecté en continu dans l'ensemble des réacteurs et il le parcourt dans un laps de temps de l'ordre de quelques semaines. Il est alors retiré du réacteur, régénéré dans une autre installation puis recyclé dans le réacteur. Toutes ces opérations s'effectuent sans que la production d'aromatiques ne soit arrêtée. Ce raccourcissement du cycle à quelques semaines permet encore d'abaisser la pression opératoire à une dizaine d'atmosphères, ce qui a, encore une fois, une influence favorable sur les rendements en aromatiques et défavorable sur l'hydrocraquage.

Dans tous ces procédés, les catalyseurs sont à base de métaux nobles sur alumine chlorée. Ils se désactivent par formation de coke qui est une espèce polyaromatique peu hydrogénée et qui limite l'accession des hydrocarbures de la charge aux sites catalytiquement actifs. L'élimination de ce coke ne peut se faire que par combustion par de faibles quantités d'oxygène. Cette opération est longue et délicate car la combustion doit être soigneusement contrôlée pour éviter les emballements thermiques. Elle doit être précédée et suivie de purges sévères pour éviter toute explosion. C'est pourquoi cette opération est faite aussi rarement que possible, malgré l'avantage que procurerait encore l'abaissement de pression au prix d'une diminution de la durée de cycle.

Ceci explique également l'avantage de travailler à basse pression en lit mobile avec régénération hors des réacteurs. Cependant la durée de cycle doit être encore au moins égale à deux semaines, car le catalyseur ne peut pas circuler trop rapidement et ceci impose que la pression opératoire ne soit pas inférieure à 8-10 bars.

D'autres types de catalyseurs très efficaces en aromatisation sont constitués de platine (entre 0,1 et 1,5 % poids), éventuellement d'un second métal tel que le rhénium, l'iridium, l'étain ou le germanium et éventuellement de soufre, le support étant constitué par un aluminosilicate cristallin zéolithique de taille de pore supérieure à 0,65 nm, compensé à plus de 90 % par des cations alcalins. Les zéolithes en question peuvent être les faujasites X et Y, la zéolithe oméga, la zéolithe ZSM 4 et la zéolithe L. Cette dernière conduit à des résultats particulièrement intéressants en aromatisation des paraffines. Comme les autres catalyseurs, ceux-ci se désactivent par formation de coke, mais nous avons constaté avec surprise qu'ils étaient facilement régénérables par l'hydrogène, ce qui permet de raccourcir considérablement la durée de la régénération et de les utiliser à très basse pression avec une amélioration spectaculaire de rendement, en utilisant un nouveau type de procédé.

L'invention consiste en un procédé de production d'hydrocarbures aromatiques à partir de coupes pétrolières contenant des paraffines.

On utilise un catalyseur contenant des métaux nobles et éventuellement du soufre déposés sur un aluminosilicate cristallin zéolithique de taille de pore supérieure à 0,65 nm, compensé à plus de 90 % par des cations alcalins. Le catalyseur est placé en lit fixe dans deux réacteurs ou ensembles de réacteurs placés en parallèle et fonctionnant à très basse pression (de 0,5 à 8 bars absolus). Lorsqu'un ensemble de réacteurs est en production d'hydrocarbures aromatiques, l'autre ensemble de réacteurs est balayé par l'hydrogène produit par le premier réacteur à la température nécessaire pour réactiver le catalyseur. Le cycle dure ainsi de 3 h à 1 semaine puis les rôles des deux ensembles sont intervertis.

Il est également possible d'utiliser plus de deux réacteurs en parallèle en répartissant à la convenance de l'utilisateur le nombre de réacteurs en opération et le nombre de réacteurs en régénération par l'hydrogène.

Les catalyseurs auxquels cette invention s'applique sont constitués de la manière suivante :

Leur support est constitué d'aluminosilicate cristallin zéolithique ou tamis moléculaire. Il est essentiel pour la déshydrocyclisation que le tamis moléculaire servant de support ait une faible acidité, ou une acidité nulle ou une faible basicité. C'est pourquoi la zéolithe devra avoir ses sites cationiques d'échanges compensés à plus de 90 % par des cations alcalins, tous autres cations introduisant une certaine acidité, soit parce qu'ils sont plurivalents et créent ainsi des sites acides, soit parce qu'ils sont réductibles ou décomposables dans les conditions de la catalyse, la réduction ou la décomposition correspondant alors à la formation de protons sur la zéolithe. Il est évident que les pores de la zéolithe alcaline ont une ouverture au moins égale aux dimensions du benzène et parmi les zéolithes utilisables on peut citer les Faujasites X et Y, la zéolithe L, la zéolithe oméga et la zéolithe ZSM 4. Ces zéolithes peuvent être utilisées sous leur forme ex synthèse, sauf les deux dernières qui contiennent des cations alkylammonium qui doivent être remplacés par des alcalins par les méthodes connues de l'homme de l'art, telles que la décomposition thermique suivie de la neutralisation par une base alcaline. On peut également échanger leurs cations de synthèse par d'autres alcalins, et les zéolithes en question peuvent donc contenir du lithium, du sodium, du potassium, du rubidium et/ou du césium.

Parmi ces zéolithes, le support préféré est la zéolithe L qui conduit à des rendements exceptionnels en aromatiques à partir de coupes aliphatiques. Cette zéolithe est synthétisée sous sa forme potassium et elle peut être utilisée telle quelle de manière économique, mais elle peut aussi contenir du sodium et surtout du rubidium ou du césium.

Le support zéolithique doit être mis en forme pour pouvoir être utilisé industriellement. Cette mise en forme sera faite soit avant, soit après de dépôt du platine, du rhénium et du soufre et on utilisera les méthodes connues de l'homme de l'art, telles que le mélange avec des liants alumine ou argile, puis l'extrusion ou la mise en forme de billes par la technique du drageoir ou de la coagulation en gouttes. Une autre technique utilisable est la mise en forme de billes ou d'extrudés d'une argile telle que le métakaolin et sa conversion en zéolithe par des techniques appropriées. On peut également utiliser la zéolithe sous forme de pastilles ou de tablettes.

Le pourcentage de platine est compris entre 0,1 et 1,5 % poids. Il peut être introduit sur le support par les méthodes décrites dans l'art antérieur telle que l'imprégnation par une solution aqueuse d'un sel ou d'un complexe de platine, comme par exemple l'acide hexachloroplatinique, le dinitro-diamminoplatine ou le chlorure de platine tétrammine. On peut également utiliser un dépôt par échange d'ions avec une solution aqueuse, d'un complexe cationique de platine tel que le chlorure de platine tétrammine.

Le catalyseur peut contenir de 0 à 1,5 % de rhénium qui est introduit sous forme de carbonyle de rhénium $Re_2(CO)_{10}$ soit par sublimation sur le support, soit par imprégnation par une solution organique. Néanmoins, il n'est pas nécessaire que le catalyseur contienne du rhénium pour que l'effet de stabilisation par l'hydrogène à haute température soit effectif.

Le catalyseur peut contenir de l'iridium, ou de l'étain ou du germanium.

Le catalyseur peut également contenir de faibles quantités de soufre. Lors de l'étape d'activation par l'hydrogène, ce soufre est vraisemblablement réduit sous forme d'acide sulfhydrique et il a alors pour rôle d'empoisonner sélectivement la fonction hydrogénolysante du catalyseur. Celui-ci contient du soufre particulièrement lorsqu'il contient du rhénium ou de l'iridium qui sont très hydrogénolysants, mais le rapport atomique soufre/Pt + autres métaux n'est pas supérieur à 1.

Une fois placé dans les réacteurs, le catalyseur doit être réduit par l'hydrogène à une température comprise entre 300 et 750 °C et de préférence entre 550 et 750 °C, ce qui a pour effet de mieux stabiliser le catalyseur.

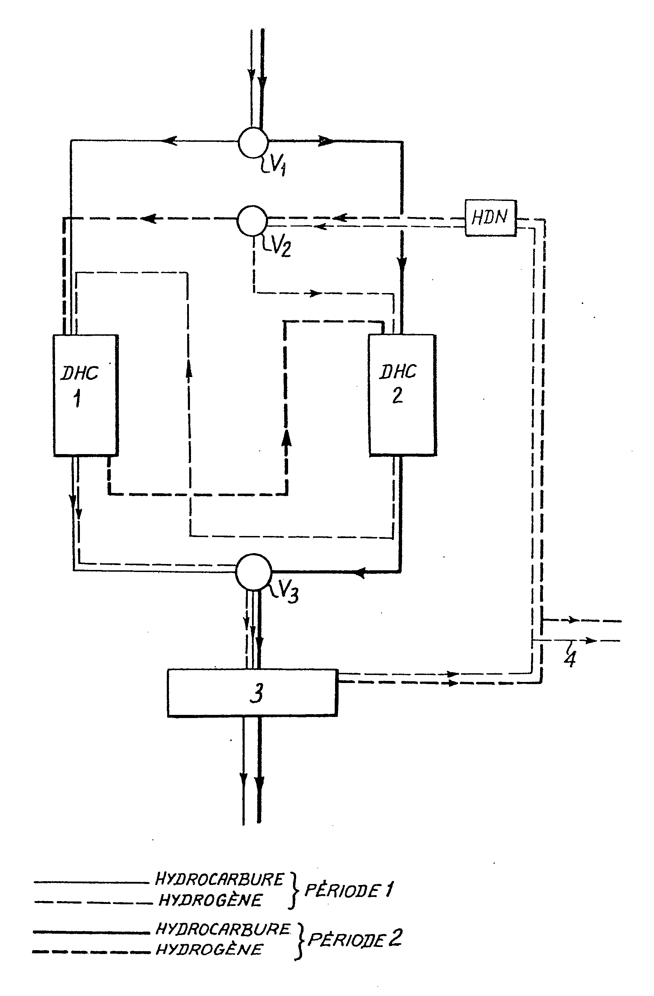

La mise en œuvre du procédé est illustrée par le schéma de la Figure 1.

Le procédé fonctionne en deux périodes alternées, la période 1 et la période 2. Sur le croquis, le cheminement des hydrocarbures (charge ou produit) est figuré en traits pleins, le cheminement de l'hydrogène est figuré en traits discontinus. Les traits sont fins pour la période 1 et épais pour la période 2. Pendant la période 1, la charge d'hydrocarbures est acheminée par l'intermédiaire d'une vanne trois voies V 1 vers le réacteur de déshydrocyclisation DHC 1 contenant le catalyseur. Elle est mélangée à de l'hydrogène de recyclage avant de pénétrer dans la section réactionnelle. Celle-ci, tout comme DHC 2 peut être constituée d'un ensemble de 1 à 4 réacteurs avec réchauffements intermédiaires de manière à compenser l'endothermicité de la réaction, comme cela est connu dans l'art antérieur. A la sortie du réacteur DHC 1 les effluents (aromatiques, charge non convertie et hydrogène) sont dirigés par une vanne trois voies V 3 vers un séparateur 3. Du fait de la faible pression opératoire, il est nécessaire d'opérer la séparation de l'hydrogène et des hydrocarbures dans de meilleures conditions qu'une simple condensation des hydrocarbures à température ambiante. En effet, l'hydrogène doit être d'une pureté supérieure à 85 % volume pour être recyclé dans les réacteurs. La séparation de l'hydrogène et des produits hydrocarburés se fait par les moyens connus de l'homme de l'art, tels que refroidissement à basse température ou compression et détente du mélange. On recueille ainsi les hydrocarbures aromatiques liquides.

L'hydrogène purifié à plus de 85 % est purgé en, 4, du débit correspondant à la production nette d'hydrogène du procédé. Le reste est dirigé sur un petit réacteur HDN (facultatif) d'hydrogénation. Ce réacteur sert à hydrogéner les traces d'oléfines contenues dans l'hydrogène, oléfines qui pourraient accélérer la désactivation du catalyseur. L'hydrogène est alors dirigé par la vanne 3 voies V 2 sur l'ensemble de réacteurs DHC 2, ce qui a pour effet de régénérer le catalyseur en place dans DHC 2. Cet hydrogène est alors recyclé vers le réacteur DHC 1 pour assurer la stabilité du catalyseur de DHC 1.

Lorsque celui-ci doit être régénéré, on passe à la période 2, pendant laquelle l'ensemble DHC 2 joue le rôle de réacteur et l'ensemble DHC 1 est régénéré par l'hydrogène provenant de DHC 2.

Bien évidemment les fours et échangeurs nécessaires n'ont pas été reportés sur ce croquis, de manière à en conserver la clarté.

Ce montage est donné à titre purement indicatif ; le procédé peut être aussi constitué de trois ou quatre ensembles de réacteurs fonctionnant suivant le même schéma, les uns en opération, les autres en régénération, selon que l'utilisateur préfère minimiser les dépenses d'achat de catalyseur ou d'investissement de réacteurs.

Les conditions opératoires de la section réactionnelle sont les suivantes :

— La pression absolue est comprise en 0,5 et 8 bars absolus en sortie de réacteur, et de préférence entre 0,5 et 3 bars. La pression entrée est supérieure, du fait de la perte de charge provoquée par le catalyseur. Grâce à cette faible pression, le rendement en aromatiques peut être augmenté notablement, tout en minimisant l'hydrocraquage. C'est ainsi qu'un catalyseur Pt-Re sur zéolithe KL produit 35 à 38 % de benzène à partir d'une coupe $C_6$ à 9 bars absolus et 500 °C. Ce même catalyseur produit 80 à 88 % poids de benzène s'il est utilisé à la pression atmosphérique à 460 °C. Parallèlement, le rendement en produits d'hydrocraquage (de méthane ou pentane) passe de 20 % à 6 % poids.

Le rapport molaire hydrogène sur hydrocarbures entrée réacteurs reste compris en 1 et 30. La température de réaction est comprise entre 400 et 500 °C. Le volume de charge liquide injecté par volume apparent de catalyseur et par heure est compris entre 0,2 et 10 h$^{-1}$. La durée de la période est comprise en 3 heures et 7 jours. Il est intéressant en fait de ne pas trop allonger la durée de la période même si le catalyseur n'est pas très désactivé, car le coke ainsi formé devient de plus en plus réfractaire à la régénération par l'hydrogène. Aussi, la durée maximale préférée de la période est de 4 jours.

Les conditions de la réactivation sont les suivantes :

— La pression est égale ou légèrement supérieure à celle de la zone réactionnelle. La température du catalyseur à réactiver est comprise entre 400 et 750 °C. L'hydrogène de réactivation provenant de la zone de séparation a une pureté supérieure à 85 % volume et son débit est égal à celui de l'hydrogène recyclé dans la zone réactionnelle.

— Le temps de réactivation est bien entendu égal à celui de la période. Malgré ces réactivations périodiques, de faibles quantités de coke réfractaire à la réactivation par l'hydrogène apparaissent petit à petit. Ce coke doit être éliminé par les méthodes classiques de régénération oxydante connues dans l'art antérieur, mais ceci n'est pas très gênant, puisque la régénération oxydante ne se fait qu'après plusieurs mois de fonctionnement du procédé.

Le procédé de l'invention est utilisable pour la production de réformats constituant d'excellentes bases carburant et pour la production de coupes aromatiques destinées à la pétrochimie. Les charges sont des essences désulfurées de distillation du pétrole brut dont le point initial est de 50 à 120 °C et le point final de 70 à 240 °C.

Une charge 50-80 °C contient essentiellement des hydrocarbures à 6 atomes de carbone et produit essentiellement du benzène. Une coupe 60-100 °C produit un mélange de benzène et de toluène. Enfin, une coupe 80-180 °C produit avec un excellent rendement un réformat de bon indice d'octane.

La présente invention sera mieux comprise à la lumière des exemples suivants donnés à titre non limitatif.

Exemple 1

20 g de catalyseur contenant 0,9 % de platine déposé sur zéolithe L échangée par des cations potassium sont placés dans un réacteur, puis réduits par un courant d'hydrogène à 600 °C pendant 7 heures. Le catalyseur est testé en déshydrocyclisation de n hexane en benzène dans les conditions suivantes : pression atmosphérique — volume de n hexane liquide injecté par volume de catalyseur et par heure (VVH) 2 h$^{-1}$. Rapport molaire hydrogène sur hydrocarbure 8. Température de réaction 460 °C. Dans ces conditions, le rendement en benzène reste stable pendant 12 h à 88 % poids alors que le rendement en produits d'hydrocraquage (de $C_1$ à $C_5$) est de 3,5 %.

Exemple 2

Après 12 h de travail du catalyseur de l'exemple 1, la charge de n hexane est coupée et on laisse l'hydrogène balayer le catalyseur à 460 °C pendant 12 h. Après ce laps de temps, la charge de n hexane est réintroduite, toujours à 460 °C. Le rendement en benzène est de 88,4 %. Après 6 h de fonctionnement la température de réaction est abaissée à 440 °C, ce qui fait chuter le rendement en aromatiques à 77,8 %. 6 h plus tard à 440 °C, le rendement est de 74,5 %, ce qui montre que le catalyseur s'est légèrement désactivé.

Exemple 3

Le catalyseur de l'exemple 2 est conservé dans le réacteur. On coupe la charge de n hexane et on laisse l'hydrogène balayer le catalyseur pendant 12 h à 460 °C. Puis la charge de n hexane est réintroduite à 460 °C pendant 6 h. Le rendement en benzène est de 88,7 %. Puis la température est descendue à 440 °C et le rendement passe à 77,8 % puis à 73,8 % après 6 h à 440 °C. Ceci montre que le catalyseur a été totalement réactivé par l'hydrogène et qu'il se désactive ensuite légèrement.

Exemple 4

Une période identique à celle de l'exemple 3 est effectuée. A 460 °C, le rendement en benzène est de 88,5 %. Après 6 h, la température est diminuée à 440 °C. Le rendement passe à 78,7 % puis 74,5 % après 6 h à 440 °C. Les exemples 1 à 4 montrent que la légère désactivation du catalyseur obtenue pendant 12 h est totalement régénérable par traitement à l'hydrogène.

Exemple 5

On utilise un catalyseur contenant 1 % de platine et 0,67 % de rhénium déposés sur zéolithe L compensée par des cations potassium. Ce catalyseur est réduit par l'hydrogène à 600 °C.

Dans les conditions de l'exemple 1, on obtient un rendement stable en benzène de 88,5 % avec 5 % de $C_1$-$C_5$ pendant 24 h. On fait subir alternativement au catalyseur 20 périodes de 24 h de réaction à 460 °C et 20 périodes de 24 h de réactivation par l'hydrogène à 460 °C. A la vingtième période de réaction, le rendement en benzène est de 86,2 % avec 3,1 % de $C_1$-$C_5$.

Cet exemple montre que les catalyseurs platine-rhénium sont aussi susceptibles d'être réactivés par l'hydrogène et qu'ils peuvent subir un grand nombre de périodes.

Exemple 6

Le catalyseur de l'exemple 1 est réduit par un courant d'hydrogène à 600 °C pendant 10 h sous 3 bars absolus, puis il est testé en déshydrocyclisation du n hexane en benzène dans les conditions suivantes : pression absolue 3 bars, VVH 3 $h^{-1}$, rapport molaire hydrogène sur hydrocarbure 8, température de réaction 525 °C. On obtient un rendement de 81,3 % de benzène avec 15 % de $C_1$-$C_5$ pendant 48 h. On fait subir alternativement au catalyseur 5 périodes de 48 h de réaction à 525 °C et 4 périodes de réactivation par l'hydrogène à 525 °C. A la cinquième période de réaction, le rendement en benzène est de 80,5 % avec 12,1 % de $C_1$-$C_5$.

**Revendications**

1. Procédé de production d'hydrocarbures aromatiques à partir de coupes pétrolières contenant des paraffines consistant à faire passer ladite charge en présence d'hydrogène, à 400 °C-550 °C sur un catalyseur contenant de 0,1 à 1,5 % poids de platine et éventuellement un métal choisi dans le groupe du rhénium, de l'iridium, de l'étain et du germanium, éventuellement du soufre en un rapport atomique soufre/métaux inférieur à 1, supportés par un alumino silicate cristallin, zéolithique, compensé par des cations alcalins, ayant une dimension de pore supérieure à 0,65 nm caractérisé en ce que le catalyseur est placé en lit fixe dans deux réacteurs ou ensembles de réacteurs, placés en parallèle fonctionnant à très basse pression de 0,5 à 8 bars absolus, de manière telle que lorsqu'un ensemble de réacteurs $DHC_1$ est en production d'hydrocarbures aromatiques, l'autre ensemble $DHC_2$ est balayé par l'hydrogène produit par le premier ensemble de réacteurs $DHC_1$ entre 400 et 750 °C pour réactiver le catalyseur, puis les rôles des deux ensembles sont inversés.

2. Procédé selon revendication 1, caractérisé en ce que le catalyseur est activé avant réaction par réduction à l'hydrogène à une température de 550 °C à 750 °C pendant une période de 1 heure à 7 jours.

3. Procédé selon les revendications 1 et 2 caractérisé en ce qu'il comporte un cycle de deux périodes, telles que :
— dans la première, la charge d'hydrocarbures mélangée à de l'hydrogène de recyclage est introduite dans l'ensemble des réacteurs de déshydrocyclisation ($DHC_1$), les effluents issus de la réaction sont dirigés dans un séparateur (3), d'où l'on extrait les produits aromatiques et purifie l'hydrogène à au moins 85 % en volume avant de le recycler, l'hydrogène purifié est alors envoyé dans la deuxième série de réacteurs de déshydrocyclisation ($DHC_2$) pour régénérer le catalyseur puis en ($DHC_1$) pour assurer la stabilité du catalyseur,
— dans la seconde, les rôles sont inversés : ($DHC_2$) agit en producteur d'hydrocarbures aromatiques tandis que le catalyseur de ($DHC_1$) est régénéré par l'hydrogène effluent de ($DHC_2$).

4. Procédé selon les revendications précédentes caractérisé en ce que : le rapport molaire Hydrogène/Hydrocarbures à l'entrée du réacteur varie de 0 à 30, le volume de charge liquide injecté par volume apparent de catalyseur et par heure est compris entre 0,2 $h^{-1}$ et 10 $h^{-1}$, la pression absolue dans la zone réactionnelle est de 0,5 à 8 bars et la température de réactivation par l'hydrogène pur au moins à 85 % en volume varie de 400 °C à 750 °C.

5. Procédé selon les revendications précédentes caractérisé en ce que la durée d'une période peut varier de 3 heures à 7 jours et est de préférence inférieure à 4 jours.

**Claims**

1. Process for the production of aromatic hydrocarbons from petroleum fractions containing paraffins, consisting in passing the said charge in the presence of hydrogen, at 400 °C to 550 °C, over a catalyst containing from 0.1 to 1.5 weight % of platinum, possibly a metal selected from the group of rhenium, iridium, tin, and germanium, and possibly sulphur in a sulphur/metal atomic ratio of less than 1, supported by a crystalline zeolithic aluminosilicate compensated by alkaline cations having a pore dimension greater than 0.65 nm, characterised in that the catalyst is located in a fixed bed within two reactors or reactor assemblies which are arranged in parallel and operate at a very low pressure of 0.5 to 8 bars absolute, in such a way that when one reactor assembly $DHC_1$ is producing aromatic hydrocarbons, the other assembly $DHC_2$ is swept by the hydrogen produced by the first reactor assembly $DHC_1$ between 400 and 750 °C to reactivate the catalyst, then the roles of the two assemblies are reversed.

2. Process according to claim 1, characterised in that the catalyst is activated before reaction by reduction with hydrogen at a temperature of 550 °C to 750 °C for a period of 1 hour to 7 days.

3. Process according to claims 1 and 2 characterised in that it includes a two stage cycle, such that :

— in the first stage, the hydrocarbon charge mixed with hydrogen being re-cycled is introduced into the dehydrocyclisation reactor assembly ($DHC_1$), the effluent from the reaction is conducted to a separator (3), where the aromatic products are extracted from it and the hydrogen is purified to at least 85 % by volume before being re-cycled, the purified hydrogen is then conducted to the second dehydrocyclisation series of reactors ($DHC_2$) to regenerate the catalyst, then into ($DHC_1$) to ensure the stability of the catalyst,

— in the second stage, the roles are reversed : ($DHC_2$) acts as aromatic hydrocarbon producer whilst the catalyst in ($DHC_1$) is regenerated by the hydrogen issuing from ($DHC_2$).

4. Process according to the preceding claims characterised in that the hydrogen/hydrocarbons molar ratio at the reactor inlet varies from 0 to 30, the volume of liquid charge injected per apparent volume of catalyst and per hour is between 0.2 $h^{-1}$ and 10 $h^{-1}$, the absolute pressure within the reaction zone is from 0.5 to 8 bars and the temperature of reactivation by the hydrogen which is at least 85 % by volume pure varies from 400 °C to 750 °C.

5. Process according to the preceding claims, characterised in that the length of a stage can vary from 3 hours to 7 days and is preferably less than 4 days.

**Ansprüche**

1. Verfahren zur Herstellung von aromatischen Kohlenwasserstoffen aus Paraffine enthaltenden Erdölfraktionen, darin bestehend, daß man das Einsatzgut in Gegenwart von Wasserstoff bei 400 °C bis 550 °C über einen 0,1-1,5 Gew.% Platin und gegebenenfalls ein Metall aus der Gruppe Rhenium, Iridium, Zinn und Germanium, gegebenenfalls auch Schwefel, bei einem Atomverhältnis von Schwefel zu Metallen von unter 1, enthaltenden Katalysator leitet, auf einem Träger aus einem durch Alkalikatione kompensiertem zeolithischen, kristallinen Tonerdesilikat mit einer Porengröße von über 0,65 nm, dadurch gekennzeichnet, daß der Katalysator als Festbettkatalysator in zwei parallel angeordneten Reaktoren oder Reaktoreinheiten angeordnet ist, die bei sehr niedrigem Druck von 0,5 bis 8 absoluten bar in der Weise arbeiten, daß während der Produktion von aromatischen Kohlenwasserstoffen in dem einen Reaktorkomplex $DHC_1$ der andere Komplex $DHC_2$ von dem im ersten Reaktorkomplex $DHC_1$ produziertem Wasserstoff bei Temperaturen von 400-750 °C gespült wird, um den Katalysator zu reaktivieren, wobei anschließend die Rollen der beiden Komplexe ausgetauscht werden.

2. Verfahren nach Anspruch 1, dadurch gekennzeichnet, daß der Katalysator vor der Reaktion durch Reduktion mit Wasserstoff bei einer Temperatur von 550 °C bis 750 °C während einer Dauer von 1 Stunde bis zu 7 Tagen aktiviert wird.

3. Verfahren nach Anspruch 1 und 2, dadurch gekennzeichnet, daß es einen Zyklus der folgenden zwei Arbeitsgänge umfaßt :

— im ersten Arbeitsgang wird das Einsatzgut von Kohlenwasserstoffen im Gemisch mit in den Kreislauf zurückgeleitetem Wasserstoff in den Dehydrozyklisationskomplex ($DHC_1$) eingeführt, die Abgase aus der Reaktion werden in einen Scheider (3) geleitet, aus dem die aromatischen Produkte gewonnen werden und in dem der mindestens 85 Vol.-% enthaltende Wasserstoff vor der Rückführung in den Kreislauf gereinigt wird, der gereinigte Wasserstoff wird daraufhin zur Regenerierung des Katalysators in eine zweite Serie von Dehydrozyklisationsreaktoren ($DHC_2$) und danach zur Gewährleistung der Katalysatorstabilität in ($DHC_1$) geleitet,

— im zweiten Arbeitsgang sind die Rollen der beiden Komplexe umgekehrt : ($DHC_2$) fungiert als Produzent der aromatischen Kohlenwasserstoffe, wohingegen der Katalysator von ($DHC_1$) durch den aus ($DHC_2$) abströmenden Wasserstoff regeneriert wird.

4. Verfahren nach den vorhergehenden Ansprüchen, dadurch gekennzeichnet, daß das Molverhältnis von Wasserstoff : Kohlenwasserstoffen bei Reaktoreintritt zwischen 0 und 30, das Volumen an flüssigem Einsatzgut, eingespritzt pro relativem Katalysatorvolumen und pro Stunde, zwischen 0,2 $h^{-1}$ und 10 $h^{-1}$, der absolute Druck bei 0,5-8 bar und die Temperatur der Reaktivierung durch den zu mindestens 85 Vol.-% reinen Wasserstoff zwischen 400 °C und 750 °C liegt.

5. Verfahren nach den vorhergehenden Ansprüchen, dadurch gekennzeichnet, daß die Dauer eines Arbeitsganges swischen 3 Stunden und 7 Tagen variieren kann und vorzugsweise unter 4 Tagen liegt.

HYDROCARBURE } PÉRIODE 1
HYDROGÈNE

HYDROCARBURE } PÉRIODE 2
HYDROGÈNE